# FASCICULE DE BREVET EUROPEEN

(11) **EP 3 046 429 B1**
(45) Date de publication et mention de la délivrance du brevet: **21.03.2018**
(21) Numéro de dépôt: 14767016.0
(22) Date de dépôt: 18.09.2014
(51) Int. Cl.: A23L 2/04, A23N 1/02, A61K 36/9066, A61K 9/00, A61Q 19/00, A61K 8/97, A61K 36/9068, A61K 9/06, A61K 9/48, A61K 36/11, A61K 36/185, A61K 36/28, A61K 36/45, A61K 36/53, A23P 30/20, A23L 33/105

(54) **OBTENTION D'UN JUS DE PLANTES FRAÎCHES PAR TRAITEMENT THERMOMECANIQUE ET SON UTILISATION EN COSMETIQUE ET THERAPEUTIQUE**
HERSTELLUNG EINES SAFTS AUS FRISCHEN PFLANZEN DURCH THERMOMECHANISCHE BEHANDLUNG SOWIE KOSMETISCHE UND THERAPEUTISCHE VERWENDUNG DAVON
OBTAINING A JUICE OF FRESH PLANTS BY THERMOMECHANICAL TREATMENT AND COSMETIC AND THERAPEUTIC USE THEREOF

(30) Priorité: 18.09.2013 FR 1358970
(43) Date de publication de la demande: 27.07.2016
(73) Titulaire: Pierre Fabre Dermo-Cosmétique, 92100 Boulogne-Billancourt (FR); Pierre Fabre Médicament, 92100 Boulogne-Billancourt (FR)
(72) Inventeur: MANDEAU, Anne, F-31200 Toulouse (FR); TALON, Christian, F-81000 Albi (FR)
(74) Mandataire: Regimbeau
(86) Numéro de dépôt international: PCT/EP2014/069942
(87) Numéro de publication internationale: WO 2015/040135

(56) Documents cités:
- WO-A1-97/33596
- WO-A2-2012/098167
- US-A- 5 403 613
- GALATHEA BISTERFELD VON MEER: "Juice from Cannabis Plants for Food/Beverage, Feed or Biogas", 9TH INTERNATIONAL CONFERENCE OF THE EUROPEAN INDUSTRIAL HEMP ASSOCIATION, 23 mai 2012 (2012-05-23), pages 1-15, XP055032562,

## Description

La présente invention concerne un procédé d'obtention d'un jus de plantes fraîches, dans lequel lesdites plantes fraîches, à l'exclusion des graines seules, sont soumises à un traitement thermomécanique en l'absence de solvant, suivi d'une opération de récupération du jus.

Un moyen largement décrit d'obtenir un jus de plantes fraîches consiste en un procédé de pressage ou encore broyage et centrifugation.

Le brevet EP0279984 décrit l'utilisation en cosmétique d'un jus de plantes de la famille de graminées obtenu suite à une extraction par écrasement, broyage et/ou concassage de la plante.

Hormis les fruits, des jus de plantes médicinales sont également réalisés (monographie EMEA-Echinacée EMEA/HMPC/104945/2006 par exemple).

Certaines techniques ont pour objectif de potentialiser l'extraction des constituants de la membrane, telles que la « Flash Détente », technique très utilisée pour le raisin, permettant d'augmenter l'extraction des anthocyanes.
Le procédé dit suspension intégrale de plantes fraîches (SIPF) permet d'obtenir des jus de plantes fraîches notamment par une étape de cryobroyage à -25°C puis -196°C et de macération de la poudre obtenue dans une solution alcoolisée.

Un traitement dit thermomécanique consiste en un traitement par énergie mécanique dans des conditions de températures particulières et adaptées. Des exemples d'énergie mécanique sont entre autres : pression, broyage, extrusion, etc.

L'extrusion est un procédé par lequel un matériau susceptible de s'écouler sous diverses conditions contrôlées est ensuite contraint à passer dans une filière à vitesse déterminée (Dziezak, J. D. (1989). Single and twin-screw extruders in food processing. Food Technol., April, 164- 174). Initialement, cette technologie a été utilisée dans l'industrie métallurgique, en Angleterre, à la fin du 18ième siècle. Quelques temps plus tard, elle a été mise en place dans l'industrie agro-alimentaire pour la fabrication de saucisson, et de pâtes alimentaires. Aujourd'hui, l'industrie alimentaire utilise abondamment cette technique d'extrusion à travers la cuisson-extrusion des produits amylacés (biscuits, biscottes, snacks, etc.) mais aussi la texturation des protéines et la fabrication d'aliments pour les animaux d'élevage ou de compagnie.

Parallèlement, la technologie d'extrusion a été largement développée pour l'industrie des thermoplastiques, et a conduit à la conception de nouvelles vis, un développement de la technologie et une ouverture vers de nouvelles applications.

Plusieurs études ont ainsi porté sur l'utilisation de l'extrudeur pour conduire des actions chimiques, mécaniques, thermomécaniques en une seule étape et en continu, comme par exemple l'extraction d'hémicelluloses (N'Diaye, S., Rigal, L., Larocque, P., Vidal, P.F., 1996. Extraction of hemicelluloses from poplar populus tremuloides, using an extruder type twin-screw reactor: A feasibility study. Bioresearch Technology 57, 61-67) (N'Diaye S., Rigal L. Factors influencing the alkaline extraction of poplar hemicelluloses in a twin-screw reactor: correlation with specific mechanical energy and residence time distribution of the liquid phase (2000) Bioresource Technology, 75 (1), pp. 13-18), de pectines (Marechal V., Rigal L. Characterization of by-products of sunflower culture - Commercial applications for stalks and heads (1999) Industrial Crops and Products, 10 (3), pp. 185-200). etc.. Dans ces cas, un solvant acide ou basique est introduit dans l'extrudeur en même temps que la matière première végétale, afin de faciliter l'extraction et la solubilisation des macromolécules recherchées (extrusion réactive).

Certaines applications sont déjà connues dans l'extraction des végétaux : la mise en oeuvre de l'extrudeur mono-vis pour l'expression d'huiles à partir de graines oléagineuses avec lequel aucun solvant n'est injecté dans le fourreau, cette extraction d'huile ne reposant que sur la compression du solide (Sriti J., Talou T., Faye M., Vilarem G. and Marzouk B. Oil extraction from coriander fruits by extrusion and comparison with solvent extraction processes. (2011) Industrial Crops and Products, 33, 659-664).

L'extrusion est également utilisée comme prétraitement, sur des marcs de fruits (pomme, cassis, canneberge..) en association avec un support solide tel que l'amidon de maïs, afin d'augmenter l'extraction des composés phénoliques (White Brittany L., Howard Luke L., Prior Ronald L.. Polyphenolic composition and antioxidant capacity of extruded cranberry pomace. (2010), J. Agric. Food Chem. 58, 4037-4042.) (Khanal RC, Howard LR, Prior RL. Procyanidin content of grape seed and pomace, and total anthocyanin content of grape pomace as affected by extrusion processing. (2009) J Food Sci, 74: H174-82).

Certains brevets mentionnent l'obtention de jus de plantes fraîches par extrusion, et entendent par extrusion une vis sans fin pour convoyer la plante avec un compresseur à piston. Les illustrations montrent un seul fourreau avec une monovis (SU1669978, SU1541071, SU1518142, SU496193, SU3986103).

Un autre brevet fait mention d'un procédé pour produire un jus à partir de plantes fraîches, avec comme prétraitement avant le pressage ou la filtration soit un broyage de la plante sous atmosphère inerte, soit un éclatement par extrusion sous vide. Cependant l'extrusion n'est ici pas le moyen d'extraire le jus, mais de préparer la plante avant extraction (EP906113).

La demande WO2012/098167 décrit l'obtention d'un jus de cannabis et son utilisation comme boisson, en citant comme exemple de procédé une extrusion à froid. L'objectif ici est de conserver au maximum les qualités nutritionnelles de la plante : acides aminés, protéines, vitamines. La pression de la plante fraîche pour l'obtention du jus est réalisée à des températures comprises entre 10 et 40°C. Il n'est donc pas mention ici de traitement thermomécanique.

L'état de la technique peut encore être complété par la citation des documents :
- US 5 403 613 (D1)
- WO97/33596 (D2)
- WO2012/098167 (D3), et
- Galathea Bisterfels von Meer, "Juice from Cannabis Plants for Food/Beverage, Feed or Biogas" (D4).

US 5 403 613 décrit un procédé de fabrication de jus de carottes par extraction à l'aide d'une solution aqueuse d'acide citrique ou de jus de citron impliquant une étape de chauffage. Le jus est ensuite récupéré par passage dans un extrudeur bi-vis contrarotatives.

WO97/33596 et WO2012/098167 décrivent d'autre procédés d'extraction de jus de diverses plantes, pour obtenir l'extrait sous forme de poudre impliquant l'addition de mono- ou di-saccharides et pour obtenir une boisson à base de cannabis, respectivement.

Enfin, « Juice from Cannabis Plants for Food/Beverage, Feed or Biogas », décrit l'extraction de jus de cannabis sans toutefois s'intéresser à un éventuel traitement thermomécanique en l'absence de solvant visant à inactiver les enzymes endogènes tout en préservant les molécules de composés d'intérêt sans aucune forme native.

Il est important de rappeler que lors du pressage de plantes fraîches, la paroi végétale freine parfois la récupération de certains composés d'intérêt, qui peuvent donc être extraits soit à l'aide d'un solvant organique, soit après traitement enzymatique. De plus, les enzymes sont facilement libérées et peuvent commencer à modifier les composés extraits dans le jus : hydrolyses, oxydations, déglycosylations, etc..

De façon surprenante et inattendue, l'adaptation d'une technique d'extrusion largement utilisée en alimentaire pour cuire et expanser des matières, à des fins d'extraction a permis de récupérer un extrait natif de la plante fraîche. Ce jus de plantes fraîches ainsi obtenu selon la présente invention peut directement être utilisé en cosmétique ou thérapeutique.

Par « extrusion » on entend selon la présente invention un traitement thermomécanique consistant à extruder la plante fraîche dans un extrudeur, de préférence un extrudeur bi-vis, associé à un traitement thermique.

Dans un mode de réalisation, l'extrusion se caractérise par le passage de la plante fraîche dans un extrudeur bivis composé de :
- une zone d'introduction des plantes fraîches : Trémie d'alimentation
- le corps principal de l'extrudeuse est constitué d'un ou plusieurs fourreaux dans lesquels tournent les vis sans fin (corotatives ou contrarotatives), ou segments de vis. De préférence, il s'agira de plusieurs fourreaux successifs adjacents. De préférence, il s'agira de deux vis sans fin corotatives. Le profil des vis pouvant varier selon la forme du filet des vis (par ex trapezoïdal, conjugué, simple ou double...) et du pas de vis. Chacune de ces vis peut également présenter différents tronçons (ou segments) qui peuvent éventuellement différer les uns des autres, par la forme du filet et/ou par le pas de vis. Eventuellement, certains des tronçons constitutifs de ces vis peuvent également correspondre à des éléments malaxeurs monolobes, ou trilobes ;
- au moins un fourreau filtrant qui :
   ✔ intervient le cas échéant pour la séparation solide/liquide ;
   ✔ comprend en outre un moyen de filtration, tel qu'une grille, et ;
   ✔ se trouve en particulier situé à la sortie de l'extrudeur ;
- des moyens de chauffage et de refroidissement car le fourreau doit être régulé en température : de 60 à 300°C.
- des moyens de pilotage de l'extrudeur tels que :
   ✔ un groupe d'entraînement : composé d'un moteur réducteur et d'un diviseur de couple, qui fournissent la puissance mécanique nécessaire à la rotation des vis ;
   ✔ automates de pilotage : permettent le suivi et la commande du procédé. Les paramètres pouvant être réglés sont : la vitesse de rotation des vis et la température de chaque fourreau.

Dans un mode de réalisation particulier, l'extrudeur est un extrudeur bi-vis à vis corotatives et copénétrantes.

Dans un autre mode de réalisation particulier de l'invention, le procédé met en oeuvre un extrudeur et de préférence un extrudeur bi-vis, à plusieurs fourreaux et terminé par un fourreau filtrant, permettant de faire varier la température et en même temps d'appliquer un cisaillement, un malaxage intense de la matière première végétale, avec pour conséquence d'entraîner un grand nombre de composés, de déstructurer la matière mais aussi d'inhiber en même temps les enzymes endogènes par un traitement thermique.

Le procédé selon l'invention consiste donc à extruder des plantes fraîches ou congelées afin d'en extraire un jus, puis à procéder à la récupération et la purification (collecte) de ce jus et enfin dans une dernière étape optionnelle, stabiliser le jus ainsi collecté.

La présente invention concerne donc un procédé d'obtention d'un jus de plantes fraîches, à l'exclusion des graines seules, soumises à un traitement thermomécanique consistant à extruder les plantes fraîches dans un extrudeur, associé à un traitement thermique permettant d'inactiver les enzymes endogènes et de conserver les molécules de composés d'intérêt sous leur forme native, en l'absence de solvant, suivi d'une opération de récupération du jus.

Selon une caractéristique de l'invention, le jus récupéré est soumis à une étape ultérieure de stabilisation, clarification et/ou filtration.

Selon une autre caractéristique de l'invention, le traitement thermomécanique consiste en une opération de trituration par cisaillement à des températures comprises entre 60°C et 300°C, de préférence entre 60°C et 120°C.

Avantageusement, le traitement thermomécanique est mis en oeuvre dans un extrudeur bi-vis comportant une première zone bi-vis corotatives et copénétrantes où se réalise la trituration desdites plantes et une seconde zone bi-vis séparées où se réalise la séparation solide/liquide. L'écoulement dans la zone bi-vis est généré par un effet de pompage et non par des forces de frottements entre vis et fourreau comme cela apparaît dans un extrudeur mono-vis.

Selon une autre caractéristique de l'invention, ladite première zone bi-vis se trouve située du côté de l'alimentation de l'extrudeur en plantes fraîches et ladite seconde zone bi-vis se trouve située du côté de la sortie de l'extrudeur.

Avantageusement, chacune desdites zones comportent au moins un fourreau et de préférence plusieurs fourreaux successifs adjacents.

Selon une caractéristique additionnelle de l'invention, les différents fourreaux comportent des moyens de commande et de contrôle de la température et des moyens de chauffage et/ou de refroidissement.

Selon une caractéristique préférentielle de l'invention, l'extrudeur bi-vis comporte au moins un fourreau filtrant.

Selon une autre caractéristique de l'invention, les moyens de chauffage sont constitués par un collier de chauffage de préférence disposé dans la première zone.

Avantageusement, l'alimentation, le transport, le cisaillement mécanique et le traitement thermomécanique permettant la trituration des plantes fraîches et l'extraction du jus sont réalisés dans la première zone de l'extrudeur, et l'opération de séparation liquide/solide est réalisée dans la deuxième zone.

De manière avantageuse, la première zone comporte plusieurs fourreaux successifs dont les températures sont réglées de façon à présenter des étages de températures croissantes échelonnées entre 60°C et 120°C, et la deuxième zone comporte au moins un fourreau porté à une température comprise entre 30°C et 120°C, de préférence entre 30°C et 100°C.

Par « plante fraîche » on entend selon la présente invention tout ou partie de plante, à l'exclusion des graines seules, utilisée fraîche ou (dé)congelée, composée de 30 à 80% d'eau, de préférence 30-90%.

Par « partie de plantes », on entendra notamment les parties aériennes telles que tiges, branches, feuilles, fruits et/ou fleurs ; et/ou les parties souterraines telles que les rhizomes, les racines et/ou les bulbes.

Dans un mode de mise en oeuvre particulier de l'invention, on utilisera les plantes entières.

Parmi les plantes utilisables dans le cadre de la présente invention, on peut citer entre autres : *Avena sativa, Melilotus officinalis, Tropaeolum majus, Echinaceae* sp., *Urtica dioica, Plantago* sp., *Erigeron canadensis, Equisetum arvense, Calendula officinalis, Melissa officinalis, Physalis* sp., *Vaccinum macrocarpon, Sambucus nigra, Zingiber officinale* et/ou *Curcuma* sp., *Betula* sp., *Mentha* sp., *Althaea* sp., les Poacées, Asteraceae et/ou Labieae et de préférence *Avena sativa, Echinaceae purpurea, Urtica dioica, Plantabo lanceolata, Eguisetum arvense.*

Dans un mode de mise en oeuvre particulier, il s'agira de : *Avena sativa* (Avoine, parties aériennes), *Melilotus officinalis* (Mélilot, parties aériennes), *Tropaeolum majus* (Capucines, parties aériennes fleuries), *Echinaceae* sp. (Echinacée, capitules floraux), *Urtica dioica* (ortie, parties aériennes), *Plantago* sp. (plantain, parties aériennes), *Erigeron canadensis* (vergerette du Canada, parties aériennes), *Equisetum arvense* (prêle, parties aériennes), *Calendula officinalis* (Calendula, fleurs), *Melissa officinalis* (Mélisse, parties aériennes), *Physalis* sp. (fruits), *Vaccinum macrocarpon* (fruits), *Sambucus nigra* (fruits et/ou fleurs), *Zingiber officinale* (Gingembre, rhizomes) *Betula* sp. (Bouleau, feuilles) et/ou *Curcuma* sp. (rhizomes).

Dans un mode de mise en oeuvre préférentiel, les plantes fraîches sont choisies dans le groupe suivant correspondant à des plantes dont les constituants actifs sont plus sensibles à la dégradation par des enzymes endogènes, tels que polyphénoloxydases, peroxydases, myrosinases, β-glucosidases, lipoxygénase :
- *Avena sativa*
- *Tropaeolum majus*
- *Echinaceae sp.*
- *Urtica dioica*
- *Plantago* sp.
- *Urtica dioica*
- *Mentha* sp.
- *Melissa officinalis*
- *Betula* sp.
- Poaceae
- Asteraceae
- Labieae.

Dans un autre mode de mise en oeuvre particulier de l'invention, il s'agira de plantules d'avoines.

Par « plantules d'avoine », on entend au sens de la présente invention l'avoine avant épiaison, c'est-à-dire au stade après germination (environ 2 semaines à 2 mois après germination) durant le stade de la montaison jusqu'à l'épiaison non comprise. On appelle «montaison» la phase de croissance qui correspond à l'élongation de la tige et à la montée de l'épi en formation, avant floraison. Des métabolites secondaires sont décrits dans la demande WO2010/054879 comme composants d'un extrait de plantule d'avoine : les flavonoïdes et les saponines de type avenacoside.

Selon un mode d'exécution de la présente invention, les plantes récoltées sont stockées de façon intermédiaire à 4°C pour le transport vers un tunnel de congélation à -40°C.

La teneur en humidité des plantes doit être au minimum de 30% pour une efficacité complète de la technique.

Ce procédé permet donc de travailler sur des plantes fraîches n'ayant subi aucune étape de séchage, conservant de fait leurs molécules natives. L'extraction est réalisée sans solvant, le procédé est très rapide, le temps de séjour de la plante dans l'extrudeur pouvant varier de quelques secondes à quelques minutes et de préférence entre 10 secondes et 5 minutes, en continu, et permet d'obtenir des débits de traitement variant en fonction de la taille de l'extrudeur de 20 à 500kg/h de plante, correspondant à l'obtention de 10 à 300l de jus/h.

Le procédé mécanique d'extrusion bi-vis entraîne la formation d'un bouchon végétal apportant une pression sur la matière ainsi qu'un éclatement des cellules, une déstructuration de la matière végétale permettant de récupérer une teneur importante en composés actifs, même peu hydrosolubles. Ceci apporte un avantage important par rapport à un pressage simple ou à une extrusion mono-vis.

De plus, les modifications de température lors de l'étape d'extrusion permettent de fluidifier le mélange marc-jus de plante et ainsi d'améliorer le rendement dans les cas où le jus est épais du fait de la présence de mucilages. Cette température appliquée au cours du procédé permet également d'inactiver des enzymes endogènes et de conserver les molécules sous leur forme native. Ceci est très important pour certains composés qui sont rapidement inactivés comme par exemple les glucosinolates dégradés par les myrosinases (crucifères), les dérivés d'acides caféiques oxydés par les polyphénols oxydases (Echinacée) (Nüsslein B., Kurzmann M., Bauer R., Kreis W. Enzymatic dégradation of Cichoric acid in Echinacea purpurea préparations (2000) J. Nat. Prod., 63, pp. 1615-1618), certaines phytoalexines activées par une déglucosidases (Avenacosides dans l'avoine)... (Morant A.V., Jorgensen K., Jorgensen C., Paquette S.M., Sanchez-Perez R., Moller B.L., Bak S. β-Glucosidases as detonators of plant chemical defense (2008) Phytochemistry, 69 (9), pp. 1795-1813).

La collecte du jus consistant à séparer le jus d'intérêt des résidus solides peut ensuite se faire par clarification et/ou filtration.

Par « clarification » on entend élimination des fragments de cellules présents dans les jus à la sortie de l'extrudeuse. Cette élimination peut se faire grâce à la technologie de clarification par effet centrifuge, qui a pour but d'éliminer le résidu solide qui pourrait colmater le média filtrant. Cette élimination peut aussi se faire directement par filtration avec un adjuvant.

Par « filtration » on entend filtration frontale ou tangentielle, où l'on peut envisager la présence d'adjuvant de filtration (type perlite, diatomées, etc..). Cette filtration permet de retenir les derniers résidus solides, le but étant d'obtenir une solution parfaitement limpide. Elle peut être suivie d'une filtration sur membrane avec un seuil de coupure défini en fonction de la taille des molécules à considérer. Elle peut également être remplacée ou suivie par une filtration sur résine ou silice, afin d'enrichir en composé d'intérêt (ex : résines d'adsorption).

Dans un mode de réalisation particulier, l'étape de clarification - filtration sera réalisée à l'aide d'un fourreau filtrant intégré en fin d'extrudeuse.

Par « stabilisation », on entend selon la présente invention :
- pour la fourniture d'un extrait liquide :
   ✔ refroidissement du jus puis congélation
   ✔ traitement des jus par filtration stérilisante sur 0.22 µm, pasteurisation, stérilisation U.H.T, ultra-filtration et conservation dans un conditionnement adapté empêchant toute contamination postérieure au traitement : poches stériles remplies sous vide, contenants stériles à usage unique.
   ✔ le stockage peut s'effectuer à température ambiante, à 4°C ou à -20°C (congélation).
   ✔ on pourra aussi envisager l'addition de conservateurs (tels que les glycols, ou l'acide sorbique, citrique, etc) ou d'alcool (minimum 15°).
- pour la fourniture d'un extrait pâteux : Concentration de façon à obtenir une teneur en matière sèche supérieure ou égale à 65%.
- pour la fourniture d'un extrait sec, les technologies de séchage sous vide, de lyophilisation ou d'atomisation peuvent être envisagées.

Les extraits obtenus, liquides, pâteux ou secs tels que définis plus haut peuvent être utilisés en l'état dans des compositions cosmétiques, pharmaceutiques ou alimentaires, destinées à être administrées par voie topique ou voie orale.

Les principaux avantages du procédé selon l'invention par rapport aux procédés existants (pressage et extrusion mono-vis) consistent en :
- l'obtention de meilleurs rendements en jus par rapport à la matière fraîche engagée (poids du jus / poids de la matière fraîche engagée) ; et/ou
- l'obtention des jus plus riches en composés ; et/ou
- l'obtention des jus contenant des molécules non dégradées par les enzymes libérées lors du froissage de la plante fraîche.

### Exemple 1

12.75 kg de parties aériennes fraîches décongelées (24h à 2°C) d'avoine (Avena sativa L.) récoltées à l'ensileuse après 2 mois de croissance (plantules d'avoine) ont été introduit dans le premier fourreau d'un extrudeur bivis à vis corotatives et copénétrantes CLEXTRAL BC45 qui en comporte 5. La température appliquée aux différents fourreaux est de 30°C/120°C/120°C/120°C/60°C.

Le schéma du procédé s'établit comme suit (durée totale de l'étape d'extrusion = 20 min ; débit de traitement 38 kg de plantes /h et 22 kg de jus /h) :

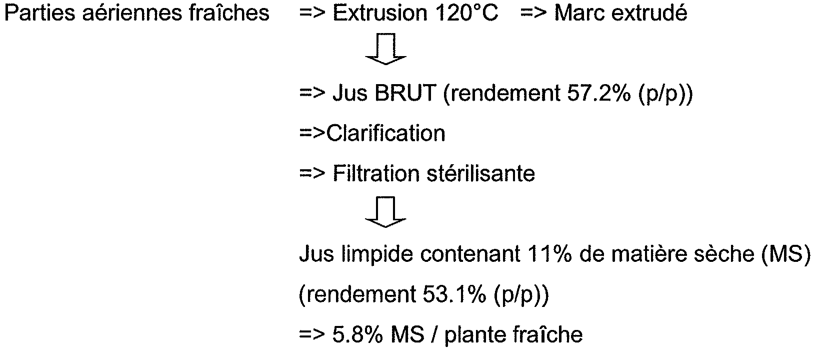

Après extrusion, on obtient 57.2% de jus p/p par rapport à la matière engagée. Des étapes de clarification et de filtration stérilisante ont ensuite été réalisées afin d'obtenir un jus limpide, avec un rendement final en jus de 53.1% contenant 11% de matière sèche, soit un rendement en matière sèche extraite de 5.8% (p/p).

Le rendement en jus obtenu par pressage (broyage - pressage - filtration) de la même matière première est de 50%, contenant 4,5% de matière sèche, soit un rendement de 2,25% (p/p).

La technologie d'extrusion permet donc d'obtenir plus de jus, et un jus plus riche en composés, et notamment en composés bioactifs. En effet, la teneur en flavonoïdes du jus obtenu selon l'exemple 1 est de 0.26%, alors qu'il n'est que de 0.02% dans le jus obtenu par pressage avec la même matière première. La teneur en flavonoïdes a donc été multipliée par 10 dans ce cas.

Nous pouvons également remarquer l'intérêt d'extruder à chaud pour la teneur en flavonoïdes : la température permet d'extraire plus de composés (dont quatre fois plus de flavonoïdes) et d'obtenir les molécules natives, non dénaturées par les enzymes.

C'est également ce que l'on remarque avec les saponines de l'avoine, les avénacosides, qui sont rapidement déglucosylées par pressage. Les molécules natives sont retrouvées seulement par traitement thermomécanique : les jus obtenus par extrusion à 120°C et 200°C contiennent bien des avenacosides (A et B) à hauteur de 89 mg et 93 mg pour 100g de matière sèche. Ils n'ont donc pas été dégradés par les desglucosidases endogènes.

| | | | | | % flavonoïdes | | | |
|---|---|---|---|---|---|---|---|---|
| Technique | Paramètres | Rdt jus* | MS | Rdt MS/plante fraîche | /MS | /jus | /MF | Avenacosides |
| | | **%** | | | | | | Desgluco avenacosides |
| Pressage | Broyage puis pressoir viticole, puis filtration | 51 | 3,78 | 1,94 | 0,44 | 0,02 | 0,01 | |
| Extrusion | 25°C | 59,70 | 7,50 | 4,47 | 0,80 | 0,06 | 0,04 | 0% |
| | 120°C | 53,13 | 11 | 5,84 | 2,40 | 0,26 | 0,15 | 89mg%g ES |
| | 200°C | 48,07 | 10 | 4,81 | 2,30 | 0,22 | 0,12 | 93mg% ES |
| Extraction H20 | 1H reflux | | | 3,10 | 1,10 | | 0,03 | |

| | | | | | | | | |
|---|---|---|---|---|---|---|---|---|
| * : après filtration | | | | | | | | |

### Exemple 2 :

3.14 kg de capitules d'Echinacée (Echinacea purpurea (L.) Moench) frais décongelés (18h à 2°C) sont introduits dans le premier fourreau d'un extrudeur bivis à vis corotatives et copénétrantes CLEXTRAL BC45 qui en comporte 5. La température appliquée aux différents fourreaux est de 100°C/100°C/100°C/100°C/60°C. Le procédé ainsi que le bilan matière sont représentés dans le tableau ci-dessous (durée totale de l'étape d'extrusion : 25min; débit de traitement 7 kg de plantes /h et 3 kg de jus /h) :

| BILAN MATIERE | PROCEDE | MAT. SECHE |
|---|---|---|
| 100 | PLANTE FRAICHE | |
| 48.1 | EXTRUSION | 16,20% |
| 26.9 | CLARIFICATION | 10,81 % |
| 25 | FILTRATION | 10,09% |

Après extrusion, nous avons donc obtenu 48.1% de jus p/p par rapport à la matière engagée. Des étapes de clarification et de filtration ont ensuite été réalisées afin d'obtenir un jus limpide, avec un rendement final en jus de 25% contenant 10.09% de matière sèche, soit un rendement en matière sèche de 2.5% (p/p).
La teneur en acides caféiques de ce jus est de :
- acide cichorique : 1.7% / matière sèche, soit 0.17% p/v
- acide caftarique : 1.21%, soit 0.12% p/v

Lorsque le jus est extrudé à température ambiante, la teneur en acide cichorique et caftarique est quasi-nulle du fait de l'action des enzymes. Lorsque le jus est obtenu par pressage des capitules frais, la teneur en ces molécules est nulle également.

En effet, les enzymes libérées lors du pressage (phénoloxydases) vont rapidement oxyder ces molécules (Nüsslein B., Kurzmann M., Bauer R., Kreis W. Enzymatic dégradation of Cichoric acid in Echinacea purpurea préparations (2000) J. Nat. Prod., 63, pp. 1615-1618, R. Bauer Standardization of Echinacea purpurea Expressed Juice with Reference to Cichoric Acid and Alkamides, Journal of herbs, Spices & Medicinal Plants Vol. 6, Iss. 3, 1999).

Lorsque l'extrusion est réalisée à température ambiante ou < à 60°C, les enzymes ne sont pas inactivées et vont dégrader les molécules d'intérêt. Dans cet exemple, seules les extrusions réalisées à 100°C ou à 200°C permettent d'extraire de la plante, sans dégradation, les acides cichoriques et caftariques (voir tableau récapitulatif).

La plupart des jus d'échinacée présents sur le marché ne contiennent pas ces molécules, seuls les extraits obtenus à l'alcool de parties aériennes sèches possèdent ces composés actifs

Nous pouvons également noter que le procédé d'extrusion, qui n'utilise comme seul solvant que l'eau naturellement présente dans la plante, permet d'extraire beaucoup plus de composés d'intérêt qu'une extraction aqueuse.

| | | | | | Exprimé/ matière sèche | | Exprimé/plante fraîche | |
|---|---|---|---|---|---|---|---|---|
| Technique Paramètres | | Rdt jus | % MS | Rdt MS/pla nte fraîche | Ac. cafta rique | Ac. cichori que | Ac. Cafta ri que | Ac. Cichori que |
| | | % | | | | | mg/g | |
| Pressage | Broyage puis pressoir viticole | 36 | 7,21 | 2,60 | 0,00 | 0,00 | 0 | 0 |
| Extrusion | 20°C | 26,7 | 8,41 | 2,24 | 0,06 | 0,04 | 0.014 | 0.009 |
| | 100°C | 25,0 | 10,09 | 2,72 | 1,21 | 1,70 | 0.33 | 0.46 |
| | 200°C | 12,46 | 12,90 | 1,61 | 1,96 | 3,61 | 0.33 | 0.61 |
| Extraction plante sèche | Eau à reflux | | | 4,73 | 0,22 | 0,05 | 0.1 | 0.023 |

### Exemple 3 :

5.11 kg de parties aériennes fraîches décongelées (20h à 2°C) de Mélisse (*Melissa officinalis* L.) sont introduits dans le premier fourreau d'un extrudeur bivis à vis corotatives et copénétrantes CLEXTRAL BC45 qui en comporte 5. La température appliquée aux différents fourreaux est de 120°C/120°C/120°C/120°C/60°C. Le procédé ainsi que le bilan matière sont représentés dans le tableau ci-dessous (durée de l'étape d'extrusion : 7 min; débit de traitement 46 kg de plantes /h et 29 kg de jus /h) :

| BILAN MATIERE | PROCEDE | MAT. SECHE |
|---|---|---|
| 100 | PLANTE FRAICHE | |
| 62.7 | EXTRUSION | |
| 49.2 | CLARIFICATION | |
| 48.8 | FILTRATION | 6,5 % |

Dans ces conditions, l'extrusion permet d'obtenir un jus avec près de 50% de rendement et contenant 6.5% de matière sèche. Cette matière contient entre autre de l'acide rosmarinique, habituellement extrait par des mélanges hydro-alcooliques tels que l'éthanol 70%. La teneur en acide rosmarinique dans la matière sèche extraite par extrusion, sans solvant organique, est ici de 2.4% (p/p), soit comparable à une extraction à l'éthanol 70 %.

### Exemple 4 :

4.5 kg de rhizomes de gingembre frais (*Zingiber officinale* Roscoe) sont introduits dans le premier fourreau d'un extrudeur bivis à vis corotatives et copénétrantes CLEXTRAL BC45. La température appliquée aux différents fourreaux est de 60°C/60°C/60°C/60°C/60°C. Le procédé ainsi que le bilan matière sont représentés dans le tableau ci-dessous :

| BILAN MATIERE | PROCEDE | MAT. SECHE |
|---|---|---|
| 100 | PLANTE FRAICHE | |
| 58.9 | EXTRUSION | |
| 50.83 | CLARIFICATION | |
| 50.8 | FILTRATION | 5,2 % |

(durée totale de l'étape d'extrusion : 5 min; débit de traitement 54 kg de plantes /h et 32 kg de jus /h)

### Exemple 5 :

5.32 kg de rhizomes de curcuma frais (*Curcuma longa L.*) sont introduits dans le premier fourreau d'un extrudeur bivis à vis corotatives et copénétrantes CLEXTRAL BC45. La température appliquée aux différents fourreaux est de 120°C/ 120°C / 120°C/ 120°C/ 120°C. le procédé ainsi que le bilan matière sont représentés dans le tableau ci-dessous (durée totale de l'étape d'extrusion : 10 min; débit de traitement 32 kg de plantes /h et 13 kg de jus /h).

| BILAN MATIERE | PROCEDE | MAT. SECHE |
|---|---|---|
| 100 | PLANTE FRAICHE | |
| 40.6 | EXTRUSION | |
| 34.6 | CLARIFICATION | 7,5 % |

Le jus obtenu n'est pas filtré afin de conserver les composés lipophiles extraits par extrusion, en suspension : la curcumine et dérivés.

Le dosage montre que leur teneur dans le jus obtenu est importante (8,36 %), supérieure à un jus présent dans la matière sèche d'un jus vendu dans le commerce (4.52 %, contenant jus de curcuma, acide citrique).

| Technique Paramètres | | Rdt jus | % MS | Curcumine (m/v) | Curcumine (m/MS) |
|---|---|---|---|---|---|
| | | % | | | |
| Jus du commerce | Congélation / décongélation / DIC* / pressage / stabilisation à l'acide citrique | | 4.01 | 0.181 | 4.52 |
| Extrusion | 120°C | 34.6 | 7.5 | 0.627 | 8.36 |

| | | | | | |
|---|---|---|---|---|---|
| * : DIC = Détente Instantanée Contrôlée | | | | | |

### Exemple 6 :

20.5 kg de parties aériennes fraîches décongelées de *Plantago lanceolata* (76% d'humidité) sont introduits dans le premier fourreau d'un extrudeur bivis à vis corotatives et copénétrantes Clextral BC45 qui en comporte 5. La température appliquée aux différents fourreaux est de 120°C. 43.8% de jus sont obtenus en sortie d'extrudeuse. Le procédé ainsi que le bilan matière sont représentés dans le tableau ci-dessous :

| PROCEDE | BILAN MATIERE | MS |
|---|---|---|
| Plante décongelée | 100 | |
| Extrusion | 45.4 | 8,11 % |
| Centrifugation | 42.3 | 7,34 % |
| Filtration AF 15 | 41.4 | |
| UF 0.3µ | 38.4 | |
| Filtration AF 140 | 36.6 | 7,11 % |
| UF 10kDa | | 6,20 % |

Une étape d'ultra-filtration a permis d'obtenir un suc de meilleure qualité organoleptique. Ce suc contient 6.2% de matière sèche. Cette matière sèche contient les actifs d'intérêt, iridoïdes (1.8%) et acides phénoliques (0.3%). Ces valeurs sont proches de celles obtenues avec un extrait hydro-alcoolique EtOH30%, et supérieures à un extrait aqueux. Nous obtenons donc un extrait sans solvant de qualité équivalente à un extrait hydro-alcoolique.

### Exemple 7:

18.8 kg de parties aériennes fraîches décongelées d'*Urtica dioica* (76% d'humidité) sont introduits dans le premier fourreau d'un extrudeur bivis à vis corotatives et copénétrantes Clextral BC45 qui en comporte 5. La température appliquée aux différents fourreaux est de 120°C. 9.4 kg de jus sont obtenus en sortie d'extrudeuse, ce qui correspond à 50% de rendement.
Ce jus, contenant 5.7% de matière sèche après centrifugation, peut être utilisé tel quel, après pasteurisation.

### Exemple 8 : gélule

| | |
|---|---|
| Jus de mélisse selon l'exemple 3, lyophilisé | 200 mg |
| Amidon | 45 mg |
| Stéarate de magnésium | 2 mg |

### Exemple 9 : crème

| | % en poids |
|---|---|
| | |
| Jus d'avoine selon l'exemple 1 | 1-5 % |
| Tribehenin PEG- 20 esters | 2-7 % |
| Isodecyl neopentanoate | 2-9 % |
| Glycérine | 0.5-10 % |
| Glycol palmitate | 1-6% |
| Cetyl alcohol | 0.5 - 3% |
| Disodium EDTA | 0.05-0.25 % |
| Conservateurs | 0.5-3 % |
| Parfum | 0.2-0.5 % |
| Xanthan gum | 0.1-0.4 % |
| Eau | qs |

## Revendications

1. Procédé d'obtention d'un jus de plantes fraîches, **caractérisé en ce que** lesdites plantes fraîches, à l'exclusion des graines seules, sont soumises à un traitement thermomécanique consistant à extruder les plantes fraîches dans un extrudeur bi-vis comportant une première zone bi-vis corotatives et copénétrantes où se réalise la trituration desdites plantes, associé à un traitement thermique permettant d'inactiver les enzymes endogènes et de conserver les molécules de composés d'intérêt sous leur forme native, en l'absence de solvant, suivi d'une opération de récupération du jus.

2. Procédé selon la revendication1, **caractérisé en ce que** ledit traitement thermique est conduit à des températures comprises entre 60°C et 300°C, de préférence entre 60°C et 120°C.

3. Procédé selon la revendication 1, **caractérisé en ce que** les plantes fraîches soumises au traitement thermomécanique sont constituées par les parties aériennes et/ou les parties souterraines de plantes fraîches, congelées ou décongelées.

4. Procédé selon l'une des revendications 1 à 3,, **caractérisé en ce que** les plantes fraîches soumises au traitement thermomécanique sont choisies parmi l'*Avena sativa, Melilotus officinalis, Tropaeolum majus, Echinaceae* sp., *Urtica dioica, Plantago* sp., *Erigeron canadensis, Equisetum arvense, Calendula officinalis, Melissa officinalis, Physalis* sp., *Vaccinum macrocarpon, Sambucus nigra, Zingiber officinale* ; *Curcuma* sp ; *Betula* sp., *Mentha* sp., *Althaea* sp., les Poacées, Asteraceae et/ou Labieae.

5. Procédé selon l'une des revendications précédentes, **caractérisé en ce que** le jus récupéré est soumis à une étape ultérieure de clarification, filtration et/ou stabilisation.

6. Procédé selon l'une des revendications 1 à 5, **caractérisé en ce que** l'extrudeur bi-vis comprend une seconde zone bi-vis où se réalise la séparation solide/liquide.

7. Procédé selon l'une des revendications 1 à 6, **caractérisé en ce que** ladite première zone bi-vis se trouve située du côté de l'alimentation de l'extrudeur en plantes fraîches.

8. Procédé selon la revendication 6, **caractérisé en ce que** ladite seconde zone bi-vis se trouve située du côté de la sortie de l'extrudeur.

9. Procédé selon l'une des revendications 1 à 8, **caractérisé en ce que** ledit extrudeur comporte au moins un fourreau et de préférence plusieurs fourreaux successifs adjacents.

10. Procédé selon la revendication 9, **caractérisé en ce que** les différents fourreaux comportent des moyens de commande et de contrôle de la température et des moyens de chauffage et/ou de refroidissement.

11. Procédé selon l'une des revendications 1 à 10, **caractérisé en ce que** ledit extrudeur bi-vis comporte au moins un fourreau filtrant.

12. Procédé selon la revendication 10, **caractérisé en ce que** les moyens de chauffage sont constitués par un collier de chauffage de préférence disposé dans la première zone.

13. Procédé selon l'une des revendications 6 à 12, **caractérisé en ce que** dans la première zone de l'extrudeur, sont réalisés l'alimentation, le transport, le cisaillement mécanique et le traitement thermomécanique permettant la trituration des plantes fraîches et l'extraction du jus et **en ce que** l'opération de séparation liquide/solide est réalisée dans la deuxième zone.

14. Procédé selon l'une des revendications 1 à 13, **caractérisé en ce que** la première zone comporte plusieurs fourreaux successifs dont les températures sont réglées de façon à présenter des étages de températures croissantes échelonnées entre 60°C et 120°C.

15. Procédé selon l'une des revendications 6 à 14, **caractérisé en ce que** la deuxième zone comporte au moins un fourreau porté à une température comprise entre 30°C et 120°C.

## Patentansprüche

1. Verfahren zur Herstellung eines Safts aus frischen Pflanzen, **dadurch gekennzeichnet, dass** die frischen Pflanzen mit Ausnahme nur der Samen einer thermomechanischen Behandlung unterzogen werden, die darin besteht, die frischen Pflanzen in einem Doppelschrauben-Extruder zu extrudieren, umfassend eine erste Zone ko-rotativer und ko-penetrierender Doppelschrauben, wo das Zermahlen der Pflanzen erfolgt, kombiniert mit einer thermischen Behandlung, die erlaubt, die endogenen Enzyme zu inaktivieren und die Moleküle von Verbindungen von Interesse in ihrer nativen Form zu konservieren, bei Abwesenheit von Lösungsmittel, gefolgt von einem Vorgang der Saftrückgewinnung.

2. Verfahren nach Anspruch 1, **dadurch gekennzeichnet, dass** die thermische Behandlung bei Temperaturen zwischen 60 °C und 300 °C, vorzugsweise zwischen 60 °C und 120 °C, inklusive durchgeführt wird.

3. Verfahren nach Anspruch 1, **dadurch gekennzeichnet, dass** die frischen Pflanzen, die der thermomechanischen Behandlung unterzogen werden, von den oberirdischen und/oder unterirdischen Teilen frischer, tiefgefrorener oder aufgetauter Pflanzen gebildet sind.

4. Verfahren nach einem der Ansprüche 1 bis 3, **dadurch gekennzeichnet, dass** die frischen Pflanzen, die der thermomechanischen Behandlung unterzogen werden, aus *Avena sativa, Melilotus officinalis, Tropaeolum majus, Echinaceae* sp., *Urtica dioica, Plantago* sp., *Erigeron canadensis, Equisetum arvense, Calendula officinalis, Melissa officinalis, Physalis* sp., *vaccinum macrocarpon, Sambucus nigra, Zingiber officinale; Curcuma* sp.; *Betula* sp., *Mentha* sp., *Althaea* sp., den Poaceae, Asteraceae und/oder Labieae ausgewählt sind.

5. Verfahren nach einem der vorangehenden Ansprüche, **dadurch gekennzeichnet, dass** der zurückgewonnene Saft einem späteren Schritt der Klärung, Filtration und/oder Stabilisierung unterzogen wird.

6. Verfahren nach einem der Ansprüche 1 bis 5, **dadurch gekennzeichnet, dass** der Doppelschraubenextruder eine zweite Doppelschraubenzone umfasst, wo die Trennung Feststoff/Flüssigkeit erfolgt.

7. Verfahren nach einem der Ansprüche 1 bis 6, **dadurch gekennzeichnet, dass** sich die erste Doppelschraubenzone an der Seite der Versorgung des Extruders mit frischen Pflanzen befindet.

8. Verfahren nach Anspruch 6, **dadurch gekennzeichnet, dass** sich die zweite Doppelschraubenzone an der Seite des Ausgangs des Extruders befindet.

9. Verfahren nach einem der Ansprüche 1 bis 8, **dadurch gekennzeichnet, dass** der Extruder mindestens einen Zylinder und vorzugsweise mehrere aufeinanderfolgende benachbarte Zylinder aufweist.

10. Verfahren nach Anspruch 9, **dadurch gekennzeichnet, dass** die verschiedenen Zylinder Steuer- und Kontrollmittel der Temperatur und Heiz- und/oder Kühlmittel aufweisen.

11. Verfahren nach einem der Ansprüche 1 bis 10, **dadurch gekennzeichnet, dass** der Doppelschraubenextruder mindestens einen Filterzylinder aufweist.

12. Verfahren nach Anspruch 10, **dadurch gekennzeichnet, dass** die Heizmittel von einer Heizschleife gebildet sind, die vorzugsweise in der ersten Zone angeordnet ist.

13. Verfahren nach einem der Ansprüche 6 bis 12, **dadurch gekennzeichnet, dass** in der ersten Zone des Extruders die Versorgung, der Transport, die mechanische Zerkleinerung und die thermomechanische Behandlung durchgeführt werden, die das Zermahlen der frischen Pflanzen und die Extraktion des Safts erlauben, und dass der Vorgang der Trennung Flüssigkeit/Feststoff in der zweiten Zone durchgeführt wird.

14. Verfahren nach einem der Ansprüche 1 bis 13, **dadurch gekennzeichnet, dass** die erste Zone mehrere aufeinanderfolgende Zylinder aufweist, deren Temperaturen derart geregelt werden, dass Stufen mit zunehmenden gestaffelten Temperaturen von zwischen 60 °C und 120 °C vorliegen.

15. Verfahren nach einem der Ansprüche 6 bis 14, **dadurch gekennzeichnet, dass** die zweite Zone mindestens einen auf eine Temperatur zwischen 30 °C und 120 °C inklusive gebrachten Zylinder aufweist.

## Claims

1. A process to obtain a juice of fresh plants, **characterized in that** said fresh plants, with the exclusion of the sole seeds, are subjected to thermomechanical treatment whereby fresh plants are extruded in a twin-screw extruder comprising a first co-rotating, co-penetrating twin-screw zone in which trituration of said plants takes place, associated with heat treatment allowing inactivation of endogenous enzymes and preserving of the molecules of compounds of interest in their native form, without use of solvent, followed by a juice recovery operation.

2. The process according to claim 1, **characterized in that** said heat treatment is conducted at temperatures between 60°C and 300°C, preferably between 60°C and 120°C.

3. The process according to claim 1, **characterized in that** the fresh plants subjected to the thermomechanical treatment are formed by the above-ground parts and/or below-ground parts of fresh plants, frozen or unfrozen.

4. The process according to one of claims 1 to 3, **characterized in that** the fresh plants subjected to the thermomechanical treatment are selected from among *Avena sativa, Melilotus officinalis, Tropaeolum majus, Echinaceae* sp., *Urtica dioica, Plantago* sp., *Erigeron canadensis, Equisetum arvense, Calendula officinalis, Melissa officinalis, Physalis* sp., *Vaccinum macrocarpon, Sambucus nigra, Zingiber officinale; Curcuma* sp; *Betula* sp., *Mentha* sp., *Althaea* sp., *Poaceae, Asteraceae* and/or *Labieae.*

5. The process according to one of the preceding claims, **characterized in that** the recovered juice is subjected to a subsequent clarification, filtration and/or stabilisation step.

6. The process according to one of claims 1 to 5, **characterized in that** the twin-screw extruder comprises a second twin-screw zone wherein solid/liquid separation takes place.

7. The process according to one of claims 1 to 6, **characterized in that** said first twin-screw zone is positioned on the fresh plant feed side of the extruder.

8. The process according to claim 6, **characterized in that** said second twin-screw zone is positioned on the outlet side of the extruder.

9. The process according to one of claims 1 to 8, **characterized in that** said extruder comprises at least one barrel and preferably several successive adjacent barrels.

10. The process according to claim 9, **characterized in that** the different barrels comprise temperature command and control means and heating and/or cooling means.

11. The process according to one of claims 1 to 10, **characterized in that** said twin-screw extruder comprises at least one filtering barrel.

12. The process according to claim 10, **characterized in that** the heating means are formed by a heating collar preferably arranged in the first zone.

13. The process according to one of claims 6 to 12, **characterized in that** feeding, conveying, mechanical shearing and thermomechanical treatment allowing trituration of the fresh plants and juice extraction take place in the first extruder zone, and **in that** the liquid/solid separating operation takes place in the second zone.

14. The process according to one of claims 1 to 13, **characterized in that** the first zone comprises several successive barrels the temperatures of which are regulated to obtain increasing temperature levels ranging from 60°C to 120°C.

15. The process according to one of claims 6 to 14, **characterized in that** the second zone comprises at least one barrel brought to a temperature of between 30°C and 120°C.
